# EUROPEAN PATENT SPECIFICATION

(11) **EP 1 439 840 B1**
(45) Date of publication and mention of the grant of the patent: **07.12.2005**
(21) Application number: 02763827.9
(22) Date of filing: 30.09.2002
(51) Int. Cl.: A61K 31/55, A61P 31/04

(54) **MONOBACTAM ANTIBACTERIAL COMPOUNDS AND METHODS OF USE THEREOF**
MONOBACTAM-ANTIBAKTERIELLE VERBINDUNGEN UND ANWENDUNGSVERFAHREN
COMPOSES ANTIBACTERIENS DE LA FAMILLE DES MONOBACTAMS ET PROCEDES D'UTILISATION

(30) Priority: 28.09.2001 US 325933 P
(43) Date of publication of application: 28.07.2004
(73) Proprietor: CHIRON CORPORATION, Emeryville California 94608-2916 (US)
(72) Inventor: SHAWAR, Ribhi, Emeryville, CA 94608-2916 (US)
(74) Representative: UEXKÜLL & STOLBERG
(86) International application number: PCT/US2002/031337
(87) International publication number: WO 2003/026669

(56) References cited:
- EP-A- 0 484 881
- WO-A-02/051356
- US-A- 4 575 496
- US-A- 5 290 929
- SCULLY B E ET AL: "USE OF AZTREONAM IN THE TREATMENT OF SERIOUS INFECTIONS DUE TO MULTIRESISTANT GRAM-NEGATIVE ORGANISMS, INCLUDING PSEUDOMONAS AERUGINOSA" AMERICAN JOURNAL OF MEDICINE, XX, XX, vol. 78, February 1985 (1985-02), pages 251-261, XP002951220 ISSN: 0002-9343

## Description

### Cross-Reference to Related Applications

This application is related to provisional application Serial No. 60/325,933, filed September 28, 2001, from which application priority is claimed under 35 USC §119(e)(1) and which application is incorporated herein by reference in its entirety.

### Field of the Invention

The present invention relates to methods of treating bacterial infections with monobactam compounds, such as PA-1806, and with a mucolytic agent, and to new compositions for the treatment of bacterial infections.

### Background of the Invention

Progressive pulmonary disease is the cause of death in over 90% of cystic fibrosis (CF) patients (Koch, C. et al., "Pathogenesis of cystic fibrosis," *Lancet* **341(8852)**:1065-9 (1993); Konstan M.W. et al., "Infection and inflammation of the lung in cystic fibrosis," Davis PB, ed., *Lung Biology in Health and Disease,* Vol. 64. New York, NY: Dekker: 219-76 (1993)). *Pseudomonas aeruginosa* is the most significant pathogen in CF lung disease. Over 80% of CF patients eventually become colonized with *P. aeruginosa* (Fitzsimmons S.C., "The changing epidemiology of cystic fibrosis," *J Pediatr* **122(1)**:1-9 (1993)). The standard therapy for *P. aeruginosa* endobronchial infections is 14 to 21 days of parenteral antipseudomonal antibiotics, typically including an aminoglycoside. However, parenteral aminoglycosides, as highly polar agents, penetrate poorly into the endobronchial space. To obtain adequate drug concentrations at the site of infection with parenteral administration, serum levels approaching those associated with nephro-, vestibulo-, and oto-toxicity are required ("American Academy of Otolaryngology. Guide for the evaluation of hearing handicap," *JAMA* **241(19)**:2055-9 (1979); Brummett R.E., "Drug-induced ototoxicity," *Drugs* **19**:412-28 (1980)).

Aerosolized administration of aminoglycosides offers an attractive alternative, delivering high concentrations of antibiotic directly to the site of infection in the endobronchial space while minimizing systemic bioavailability (Touw D.J. *et al.,* "Inhalation of antibiotics in cystic fibrosis," *Eur Respir J* **8**:1594-604 (1995); Rosenfeld M. *et al.,* "Aerosolized antibiotics for bacterial lower airway infections: principles, efficacy, and pitfalls," *Clinical Pulmonary Medicine* **4(2)**:101-12 (1997)).

Tobramycin is commonly prescribed for the treatment of serious infections with *P. aeruginosa.* It is an aminoglycoside antibiotic produced by the actinomycete, *Streptomyces tenebrarius.* Low concentrations of tobramycin (< 4 µg/mL) are effective in inhibiting the growth of many Gram-negative bacteria and under certain conditions may be bactericidal (Neu, H.C., "Tobramycin: an overview," *J Infect Dis* 134, Suppl: S3-19 (1976)). Tobramycin is poorly absorbed across mucosal surfaces, conventionally necessitating parenteral administration. Tobramycin activity is inhibited by purulent sputum: high concentrations of divalent cations, acidic conditions, increased ionic strength and macromolecules that bind the drug all inhibit tobramycin in this environment. It is estimated that 5 to 10 times higher concentrations of tobramycin are required in the sputum to overcome these inhibitory effects (Levy J. et al., "Bioactivity of gentamicin in purulent sputum from patients with cystic fibrosis or bronchiectasis: comparison with activity in serum," *J Infect Dis* **148(6)**:1069-76 (1983); Mendelman, P.M., et al., "Aminoglycoside penetration, inactivation, and efficacy in cystic fibrosis sputum," *Am. Rev. Respir. Dis.* **132:**761-765 (1985)).

A preservative-free, stable, and convenient formulation of tobramycin (TOBI® tobramycin solution for inhalation; 60 mg/mL tobramycin in 5 mL of 1/4 normal saline) for administration via jet nebulizer was developed by PathoGenesis Corporation, Seattle, Washington (now Chiron Corporation). The combination of a 5 mL BID TOBI dose (300 mg tobramycin) and the PARI LC PLUS/PulmoAide compressor delivery system was approved under NDA 50-753, December 1997, for the management of *P. aeruginosa* in CF patients, and remains the industry standard for this purpose. The aerosol administration of a 5ml dose of a formulation containing 300 mg of tobramycin in quarter normal saline for the suppression of *P.* *aeruginosa* in the endobronchial space of a patient is disclosed in U.S. Patent No. 5,508,269, the disclosure of which is incorporated herein in its entirety by this reference.

Another factor tending to inhibit the effectiveness of aerosolized antibiotic agents in the treatment of CF patients is the presence of an abnormally high level of mucous secretions. N-acetylcysteine is an aerosolized mucolytic agent often used as adjunctive therapy for pulmonary complications of cystic fibrosis (CF) in combination with vigorous chest physiotherapy. The viscosity of mucous secretions in the lungs is dependent upon the concentrations of mucoprotein, the presence of disulfide bonds between these macromolecules and DNA. N-acetylcysteine acts to split the sulfide bonds in the macromolecules thereby decreasing viscosity, allowing for removal by normal chest physiology. The action of N-acetylcysteine is pH dependent - mucolytic action is significant at ranges of pH 7-9.

An additional factor that contributes to viscous mucus in CF patients is extracellular DNA. Bacterial cell death and subsequent cell lysis releases DNA into the extracellular environment. This high extracellular DNA content works to further thicken airway secretions. (Duplantier D, McWaters DS. Cystic fibrosis: progress against a childhood killer. *US Pharm* 1992; 17:34-52.). Recombinant human DNase has been demonstrated to reduce the viscosity of sputum in CF patients by hydrolyzing the extracellular DNA. DNase is a highly purified solution of recombinant human deoxyribonuclease I (rhDNase), an enzyme that selectively cleaves DNA. (Kastrup EK, et al. (eds) Respiratory inhalant products. In *Drug Facts and Comparisons,* 1998. St. Louis, Facts and Comparisons. pp 1141-1163.) Studies have demonstrated that daily administration of recombinant human DNase resulted in improvement in pulmonary function, as assessed by FEV1, above baseline. Recombinant human DNase use also resulted in significant reduction in the number of patients experiencing respiratory tract infections that required the use of parenteral antibiotics. (Ranasinha C, et al., Efficacy and safety of short-term administration of aerosolized recombinant human DNase I in adults with stable stage cystic fibrosis. *Lancet* 1993; **342:**199-202.; Genentech. PULMOZYME® package insert. San Francisco, CA, December,1993).

Although the some of the current conventional delivery systems and antibacterials have been shown to be clinically efficacious, there is a need for new and improved methods, compositions and devices for the delivery of antibiotic compounds to a patient by inhalation to reduce administration costs, increase patient compliance and enhance overall effectiveness of the inhalation therapy.

### Summary of the Invention

In accordance with the present invention, it has now been discovered that patients suffering from a pathogenic endobronchial microbial infection can be advantageously treated by administering to the subject a therapeutically effective amount of an antibacterial monobactam compound, or a pharmaceutically acceptable salt or prodrug thereof, and administering to the subject an amount of a mucolytic agent effective to enhance the antibacterial activity of the monobactam compound in the endobronchial space of the patient The mucolytic agent may be administered to the patient prior to, simultaneously with, or after administration of the antibacterial monobactam compound. The mucolytic agent may also be administered by the same or a different route of administration for the antibacterial monobactam compound. For example, the mucolytic agent may be administered orally and the antibacterial monobactam compound administered via inhalation or the mucolytic agent may also be administered via inhalation.

In other aspects, the present invention provides new compositions for patients suffering from a pathogenic endobronchial microbial infection, comprising an antibacterially effective amount of monobactam compound, or a pharmaceutically acceptable salt or prodrug thereof, and an amount of a mucolytic agent effective to enhance the antibacterial activity of the monobactam compound when administered to the endobronchial space of a patient.

In a further aspect, this invention provides a therapeutic package suitable for commercial sale, comprising a container and a therapeutically effective amount of an antibacterial monobactam compound, or a pharmaceutically acceptable salt or prodrug thereof, and an amount of a mucolytic agent effective to enhance the antibacterial activity of the monobactam compound in the endobronchial space of the patient. In one embodiment of the package, the antibacterial monobactam compound is [2R [2α, 3α(Z)]]-3-[[[[1 (2 amino-4-thiazolyl)-2 [(2 methyl 4-oxo-1 sulfo-3 azetidinyl)amino]-2 oxoethylidene]amino]oxy]methyl]-6,7-dihydroxy-2-quinoxalinecarboxylic acid, aztreonam or carumonam, or a pharmaceutically acceptable salt or prodrug thereof. In a further embodiment of a package, the mucolytic agent is selected from the group consisting of N-acetyl-L-cysteine, recombinant human Dnase, Erdosteine, Gauifenesin, and sodium taurocholate. The package may optionally be associated with written matter instructing that the mucolytic agent be administered prior to administration of the antibacterial monobactam compound. The therapeutic package suitable for commercial sale may optionally further comprise a nebulizer for administration of the antibacterial monobactam compound and, optionally, a nebulizer for administration of the mucolytic agent

### Brief Description of the Drawings

The foregoing aspects and many of the attendant advantages of this invention will become more readily appreciated as the same become better understood by reference to the following detailed description, when taken in conjunction with the accompanying drawings, wherein:
FIGURE 1 is a graph showing the time kill kinetics of PA-1806 against *P. aeruginosa* ATCC 27853 at a concentration of 0 µg/ml - control (◆), 0.5 µg/ml (■), 1 µg/ml (▲), 2 µg/ml (■), and 4 µg/ml (*) as described in Example 1;
FIGURE 2 is a graph showing the time kill kinetics of PA-1806 against a *B. cepacia* clinical isolate at a concentration of 0 µg/ml - control (◆), 1 µg/ml (■) and 8 µg/ml (▲), as described in Example 1;
FIGURE 3 is a graph showing the time kill kinetics of PA-1806 against *P. aeruginosa* ATCC 27853 in the presence and absence of mucin at a concentration of 0 µg/ml control, no mucin (◆), 0 µg/ml - control, mucin (■), 2 µg/ml - no mucin (▲), 4 µg/ml - no mucin (x), 8 µg/ml -mucin (*), 64 µg/ml -mucin (●), and 128 µg/ml -mucin (+), as described in Example 1;
FIGURE 4 is a graph showing the time kill kinetics of tobramycin against *P. aeruginosa* ATCC 27853 in the presence and absence of mucin at a concentration of 0 µg/ml control, no mucin (◆), 0 µg/ml - control, mucin (■), 0.5 µg/ml tobramycin (▲), 12.5 µg/ml tobramycin (*), 25 µg/ml tobramycin (*), and 50 µg/ml (●), as described in Example 1.

### Detailed Description of the Preferred Embodiment

In accordance with the present invention, it has now been discovered that patients suffering from a pathogenic endobronchial microbial infection can be advantageously treated by administering to the patient a therapeutically effective amount of an antibacterial monobactam compound, or a pharmaceutically acceptable salt or prodrug thereof, and administering to the patient an amount of a mucolytic agent effective to enhance the antibacterial activity of the monobactam compound in the endobronchial space of the patient The mucolytic agent may be administered to the patient prior to, simultaneously with, or after administration of the antibacterial monobactam compound. In a presently preferred embodiment of the invention, the mucolytic agent is administered to the patient prior to administration of the antibacterial monobactam compound.

In other aspects, the present invention relates to methods for inhibiting the growth of susceptible microbes in vitro in the presence of mucin by treating the microbes with a antibacterially effective amount of an monobactam compound, or a pharmaceutically acceptable salt or prodrug thereof, and with an amount of a mucolytic agent effective to enhance the antibacterial activity of the monobactam compound by removing or reducing the interfering or antagonistic effect of mucin.

In other aspects, the present invention provides pharmaceutical compositions for patients suffering from a pathogenic endobronchial microbial infection, comprising an antibacterially effective amount of monobactam compound, or a pharmaceutically acceptable salt or prodrug thereof, and an amount of a mucolytic agent effective to enhance the antibacterial activity of the monobactam compound when administered to the endobronchial space of a patient

Monobactam compounds useful in the practice of the invention have antibacterial activity against a wide range of organisms, and preferably have activity against gram negative bacteria, such as *Pseudomonas aeruginosa, Stenotrphomonas maltophilia* and *Burkholderia cepacia* (Fung-Tomc, J. et al., "Antibacterial Activity of BMS-180806 A New Catechol Containing Monobactam, Antimicrobial Agents and Chemotherapy" 41:1010-1016 (1997). Representative monobactam compounds for this purpose are disclosed in U.S. Patent No. 5,290,929, the disclosure of which is hereby incorporated herein by this reference. A presently particularly preferred monobactam compound for use in the practice of the invention is [2R [2α, 3α(Z)]]-3-[[[[1 (2 amino-4-thiazolyl)-2 [(2 methyl 4-oxo-1 sulfo-3 azetidinyl)amino]-2 oxo-ethylidene]amino]oxy]methyl]-6,7-dihydroxy-2-quinoxalinecarboxylic acid, also known as PA-1806, as disclosed in U.S. Patent No. 5,290,929. PA-1806 has the following structure: 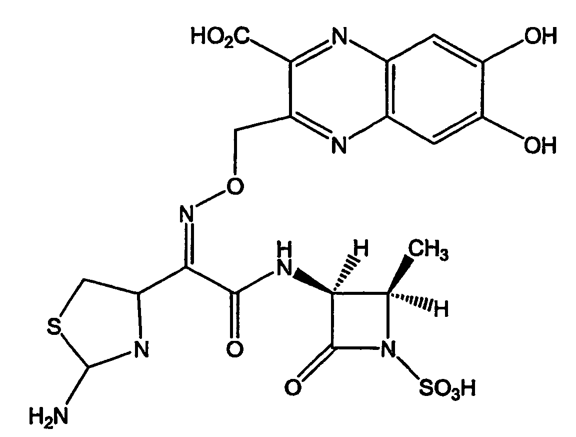 Although PA-1806 is presently preferred, other salts, prodrug and antibacterially effective monobactam derivatives, such as aztreonam and carumonam, for example, may be used in the practice of the invention.

As used herein, the term "mucolytic agent" means any agent that breaks down, or hydrolyzes or liquefies mucus or mucopolysaccharides mucus sufficiently to enhance the antibacterial effect of the monobactam compounds of the composition. Suitable mucolytic agents can include, without limitation, N-acetyl-L-cysteine (MUCOSIL™; Dey Laboratories), recombinant human DNase (PULMOZYME®, Genentech, Inc.), Erdosteine (expectorant that enhances airway secretion and thus reduces viscosity of mucous), Gauifenesin (an approved expectorant), and sodium taurocholate (for *in vitro* use). Those knowledgeable in the art will be able to determine the proper effect amount of the mucolytic agent administered to the patient. Examples of dosage ranges of mucolytic agents include Pulmozyme (Dornase alpha): 2.5 mg single use ampoule administered once daily by nebulization, Mucofilin (acetylcysteine), 20% solution (200 mg/mL) with 3 to 5 ml of the 20% solution for 3 to 4 times a day administered by nebulization. Expectrorin (guaifenesin): 1-2 tablets administered orally every 12 hours for up to 2400 mg. The mucolytic agent may be administered prior to the monobactam compound and allowed to take effect, such as by waiting a sufficient period of time after administration such as about two to five minutes, or may be administered simultaneously with or after the monobactam compound, as is hereinafter further described.

The compounds of the present invention can be used in the form of salts derived from inorganic or organic acids. These salts include but are not limited to the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-napthalenesulfonate, oxalate, pamoate, pectinate, persulfate, 3-phenylproionate, picrate, pivalate, propionate, succinate, tartrate, thiocyanate, p-toluenesulfonate and undecanoate. Also, the basic nitrogen-containing groups can be quaternized with such agents as loweralkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl and stearyl chlorides, bromides and iodides, aralkyl halides like benzyl and phenethyl bromides, and others. Water or oil-soluble or dispersible products are thereby obtained.

Examples of acids which may be employed to form pharmaceutically acceptable acid addition salts include such inorganic acids as hydrochloric acid, sulphuric acid and phosphoric acid and such organic acids as oxalic acid, maleic acid, succinic acid and citric acid. Basic addition salts can be prepared *in situ* during the final isolation and purification of the compounds of formula (I), or separately by reacting carboxylic acid moieties with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine and the like.

The compounds of the present invention can be used in the form of pharmaceutically acceptable prodrugs. The term "pharmaceutically acceptable prodrugs" as used herein refers to those prodrugs of the compounds of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed *in vivo* to yield the parent compound of the above formula, for example by hydrolysis in blood. A thorough discussion is provided in T. Higuchi and V. Stella, Pro-drugs as Novel Delivery Systems, Vol. 14 of the A.C.S. Symposium Series, and in Edward B. Roche, ed., Bioreversible Carriers in Drug Design, American Pharmaceutical Association and Pergamon Press, 1987, both of which are incorporated herein by reference.

The compounds of the invention are useful *in vitro* in inhibiting the growth of Gram negative pathogenic microbes, and *in vivo* in human and animal hosts for treating Gram negative pathogenic microbial infections, including infections of Acinetobacter Spp., Aeromona Spp., *Alcaliginenes xylosoxidans, B. cepacia,* Citrobacter Spp., Enterobacter Spp., *Escherichia coli, Haemophilus influenzae,* Klebsiella Spp., *Moraxella catarhalis,* Morganella Spp., Neisseria Spp., Proteus Spp., Providencia Spp., *Pseudomonas aeruginosa,* Salmonella Spp., Serratia spp., Shigella Spp., *Stenotrophomonas maltophilia,* and Yersinia Spp. The compounds may be used alone or in compositions together with a pharmaceutically acceptable carrier.

Total daily dose administered to a host in single or divided doses may be in amounts, for example, from 0.001 to 1000 mg/kg body weight daily and more preferred from 1.0 to 30 mg/kg body weight daily. Dosage unit compositions may contain such amounts of submultiples thereof to make up the daily dose.

The amount of active ingredient that may be combined with the carrier materials to produce a single dosage form will vary depending upon the host treated and the particular mode of administration.

It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy.

The compounds of the present invention may be administered orally, parenterally, sublingually, by aerosolization or inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or ionophoresis devices. The term parenteral as used herein includes subcutaneous injections, intravenous, intramuscular, intrasternal injection, or infusion techniques.

Injectable preparations, for example, sterile injectable aqueous or oleagenous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols, which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

The compounds of the present invention can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott, Ed., *Methods in Cell Biology,* Volume XIV, Academic Press, New York, N.W., p. 33 *et seq* (1976).

In other aspects, the current invention concerns concentrated monobactam formulations, such as concentrated formulations of -[[[[1 (2 amino-4-thiazolyl)-2 [(2 methyl 4-oxo-1 sulfo-3 azetidinyl)amino]-2 oxoethylidene]amino]oxy]methyl]-6,7-dihydroxy-2-quinoxalinecarboxylic acid (PA-1806), suitable for efficacious delivery of the monobactam by aerosolization into endobronchial space. This aspect of the invention is most preferably suitable for formulation of concentrated monobactams for aerosolization by jet, vibrating porous plate or ultrasonic nebulizers to produce average monobactam aerosol particle sizes between 1 and 5 µ necessary for efficacious delivery of the monobactams into the endobronchial space to treat infections by susceptible Gram negative organisms, such as, for example, Acinetobacter Spp., Aeromona Spp., *Alcaliginenes xylosoxidans, B. cepacia,* Citrobacter Spp., Enterobacter Spp., *Escherichia coli, Haemophilus influenzae,* Klebsiella Spp., *Moraxella catarhalis,* Morganella Spp., Neisseria Spp., Proteus Spp., Providencia Spp., *Pseudomonas aeruginosa,* Salmonella Spp., Serratia spp., Shigella Spp., *Stenotrophomonas maltophilia,* and Yersinia Spp. The formulation contains minimal yet efficacious amounts of a monobactam of the invention formulated in the smallest possible volume of physiologically acceptable solution having a salinity, or dry powder, adjusted to permit generation of monobactam aerosol well-tolerated by patients but preventing the development of secondary undesirable side effects such as bronchospasm and cough.

Primary requirements for any aerosolized formulation are its safety and efficacy. Additional advantages are lower treatment cost, practicality of use, long-shelf life, storage and optimization of nebulizer.

The aerosol formulations of the invention are nebulized predominantly into particle sizes, which can be delivered to the terminal, and respiratory bronchioles where the susceptible bacteria reside in patients with chronic bronchitis and pneumonia. Many susceptible bacteria are present throughout in airways down to bronchi, bronchioli and lung parenchema. However, it is most predominant in terminal and respiratory bronchioles. During exacerbation of infection, bacteria can also be present in alveoli. It is therefore clear that any therapeutic formulation must be delivered throughout the endobronchial tree to the terminal bronchioles and eventually to the parenchymal tissue.

Aerosolized monobactam formulations are formulated for efficacious delivery of a monobactam compound of the invention to the lung endobronchial space. A specific jet, vibrating porous plate, mechanical or gas-propelled droplet extrusion, or ultrasonic nebulizer is selected to allow the formation of monobactam aerosol particles having with a mass medium average diameter predominantly between 1 to 5 µ. The formulated and delivered amount of the monobactam is efficacious for treatment and prophylaxis of endobronchial infections, particularly those caused by susceptible Gram negative organisms, such as, for example, Acinetobacter Spp., Aeromona Spp., *Alcaliginenes xylosoxidans, B. cepacia,* Citrobacter Spp., Enterobacter Spp., *Escherichia coli, Haemophilus influenzae,* Klebsiella Spp., *Moraxella catarhalis,* Morganella Spp., Neisseria Spp., Proteus Spp., Providencia Spp., *Pseudomonas aeruginosa,* Salmonella Spp., Serratia spp., Shigella Spp., *Stenotrophomonas maltophilia,* and Yersinia Spp. The formulation has salinity adjusted to permit generation of monobactam aerosol well tolerated by patients. Further, the formulation has balanced osmolarity ionic strength and chloride concentration. The formulation has a smallest possible aerosolizable volume able to deliver effective dose of a monobactam of the invention to the site of the infection. Additionally, the aerosolized formulation does not impair negatively the functionality of the airways and does not cause undesirable side effects.

Aerosolized monobactam formulations according to the invention contain from 10-150 mg, preferably 30 mg, of a monobactam antibiotic drug per 1 mL of aqueous solution, or 10-150, preferably 30 mg of a monobactam antibiotic drug per 1 mL of the quarter normal saline. This corresponds to amounts of monobactam that are minimal yet efficacious amounts of monobactam to suppress infections in the endobronchial space caused by susceptible Gram negative organisms, such as, for example, Acinetobacter Spp., Aeromona Spp., *Alcaliginenes xylosoxidans, B. cepacia,* Citrobacter Spp., Enterobacter Spp., *Escherichia coli, Haemophilus influenzae,* Klebsiella Spp., *Moraxella catarhalis,* Morganella Spp., Neisseria Spp., Proteus Spp., Providencia Spp., *Pseudomonas aeruginosa,* Salmonella Spp., Serratia spp., Shigella Spp., *Stenotrophomonas maltophilia,* and Yersinia Spp.

Presently preferred aerosol monobactam formulations according to the invention contain 10-150 mg of monobactam per 1 mL of the quarter normal saline (corresponding to 10-150 mg/mL of monobactam that is minimal yet efficacious amount of monobactam to suppress the bacterial infections in endobronchial space), or corresponding amounts of monobactam formulated as a dry powder for aerosol delivery.

Both patients and aerosol generating devices are sensitive to the osmolarity, pH, and ionic strength of the formulation. It has now been discovered that this problem is conveniently solved by formulating some monobactam solutions in quarter normal saline, that is saline containing 0.225% of sodium chloride, and that ¼ N saline is a suitable vehicle for delivery of monobactam into the endobronchial space.

Chronic bronchetic patients and other patients with chronic endobronchial infections have a high incidence of bronchospastic or asthmatic airways. These airways are sensitive to hypotonic or hypertonic aerosols, to the presence of a permanent ion, particularly a halide such as chloride, as well as to aerosols that are acidic or basic. The effects of irritating the airways can be clinically manifested by cough or bronchospasm. Both of these conditions can prevent efficient delivery of aerosolized monobactam into the endobronchial space.

In certain aspects, the aerosolized monobactam formulations of the invention ¼ NS with 10-150 mg of monobactam per ml of ¼ NS have an osmolarity in the range of 130-550 mOsm/L. This is within the safe range of aerosols administered to a chronic bronchitis patient.

The pH of the aerosolized formulations of the invention is also important for aerosol delivery. When the aerosol is either acidic or basic, it can cause bronchospasm and cough. The safe range of pH is relative; some patients will tolerate a mildly acidic aerosol that in others will cause bronchospasm. Any aerosol with a pH of less than 4.5 usually will induce bronchospasm in a susceptible individual; aerosols with a pH between 4.5 and 5.0 will occasionally cause this problem. An aerosol with a pH between 5.0 and 8.4 is considered to be safe. The optimum pH for the aerosol formulation was determined to be between pH 7.0 and 8.4.

The aerosolized formulations of the invention are nebulized predominantly into particle sizes allowing a delivery of the drug into the terminal and respiratory bronchioles and lower airways and tissues where the bacteria reside. For efficacious delivery of the monobactam of the invention to the lung endobronchial space of airways in an aerosol, the formation of aerosol particles having a mass medium average diameter predominantly between 1 to 5 µ is necessary. The formulated and delivered amount of monobactam for treatment and prophylaxis of endobronchial infections, particularly those caused by susceptible Gram negative organisms, such as, for example, Acinetobacter Spp., Aeromona Spp., *Alcaliginenes xylosoxidans, B. cepacia,* Citrobacter Spp., Enterobacter Spp., *Escherichia coli, Haemophilus influenzae,* Klebsiella Spp., *Moraxella catarhalis,* Morganella Spp., Neisseria Spp., Proteus Spp., Providencia Spp., *Pseudomonas aeruginosa,* Salmonella Spp., Serratia spp., Shigella Spp., *Stenotrophomonas maltophilia,* and Yersinia Spp., must effectively target the lung surface. The formulation must have a smallest possible aerosolizable volume able to deliver effective dose of monobactam to the site of the infection. The formulation must additionally provide conditions that would not adversely affect the functionality of the airways. Consequently, the formulation must contain enough of the drug formulated under the conditions, which allow its efficacious delivery, while avoiding undesirable reactions. The new formulations according to the invention meet all these requirements.

The formulated dose of monobactam of 10-150 mg/mL of one-quarter diluted saline at pH 7.5-8.0 has been found to be optimal for the most efficacious delivery. Although in some instances both lower and higher doses, typically from 1-200 mg/mL may be advantageously used, the 30-150 mg/mL dose of monobactam is preferred.

According to this aspect of the invention, monobactam is formulated in a dosage form intended for inhalation therapy by patients with chronic bronchitis and pneumonia. Since the patients reside throughout the world, it is imperative that the formulation has reasonably long shelf life. Storage conditions and formulation stability thus become important

As discussed above, the pH of the solution is important. A pH between 5.0 and 8.4, preferably about 6.5, is optimal from the storage and longer shelf-life point of view.

The formulation is typically stored in a one-milliliter low-density polyethylene (LDPE) vials. The vials are aseptically filled using a blow-fill-seal process. The vials are sealed in foil overpouches.

Stability of the formulation with respect to oxidation is another very important issue. If the drug is degraded before aerosolization, a smaller amount of the drug is delivered to the lung, thus impairing the treatment as well as provoking conditions that could lead to the development of resistance to monobactam, because the delivered dose would be too small. Moreover, monobactam degradation products may provoke bronchospasm and cough. To prevent oxidative degradation of monobactam and in order to provide acceptable stability, a product with low oxygen content is produced by packaging the LDPE vials in oxygen-protective packaging comprising foil overpouches, six vials per overpouch. Prior to vial filling, the solution in the mixing tank is nitrogen sparged and the annular overpouch headspace is nitrogen purged. In this way, both hydrolysis and oxidation of monobactam is prevented.

Another important part of this aspect of the invention is an aerosolization device, such as a jet, vibrating porous plate, mechanical or gas-propelled droplet extrusion, or ultrasonic nebulizer, that is able to nebulize the formulation of the invention into aerosol particle size predominantly in the size range from 1-5 µ. Predominantly in this application means that at least 70% but preferably more than 90% of all generated aerosol particles are within 1-5 µ range.

Nebulizers such as jet, ultrasonic, vibrating porous plate, and energized dry powder inhalers, that can produce and deliver particles between the 1 and 5 µm particle size that is optimal for treatment of susceptible Gram negative organisms, such as, for example, Acinetobacter Spp., Aeromona Spp., *Alcaliginenes xylosoxidans, B. cepacia,* Citrobacter Spp., Enterobacter Spp., *Escherichia coli, Haemophilus influenzae,* Klebsiella Spp., *Moraxella catarhalis,* Morganella Spp., Neisseria Spp., Proteus Spp., Providencia Spp., *Pseudomonas aeruginosa,* Salmonella Spp., Serratia spp., Shigella Spp., *Stenotrophomonas maltophilia,* and Yersinia Spp. infections, are currently available or under development. A jet nebulizer works by air pressure to break a liquid solution into aerosol droplets. Vibrating porous plate nebulizers work by using a sonic vacuum produced by a rapidly vibrating porous plate to extrude a solvent droplet through a porous plate. An ultrasonic nebulizer works by a piezoelectric crystal that shears a liquid into small aerosol droplets.

While a variety of devices are available, only a limited number of these nebulizers are suitable for the purposes of this aspect of the invention. Preferred nebulizers useful in the present invention include, for example, AeroNeb™ and AeroDose™ vibrating porous plate nebulizers (AeroGen, Inc., Sunnyvale, California), Sidestream® nebulizers (Medic-Aid Ltd., West Sussex, England), Pari LC® and Pari LC Star® jet nebulizers (Pari Respiratory Equipment, Inc., Richmond, Virginia), and Aerosonic™ (DeVilbiss Medizinische Produkte (Deutschland) GmbH, Heiden, Germany) and UltraAire® (Omron Healthcare, Inc., Vernon Hills, Illinois) ultrasonic nebulizers.

While the compounds of the invention can be administered as the sole active pharmaceutical agent, they can also be used in combination with one or more other agents having a different antimicrobial spectrum used in the treatment of other pathogenic microbial infections. Representative agents useful in combination with the compounds of the invention for the treatment of *M*. *tuberculosis* include, for example, isoniazid, rifampin, pyrazinamide, ethambutol, rifabutin, streptomycin, ciprofloxacin and the like. Representative agents useful in combination with the compounds of the invention for the treatment of *Clostridium* include, for example, vancomycin, metronidazole, bacitracin and the like. Representative agents useful in combination with the compounds of the invention for the treatment of *Cryptosporidium* include, for example, furoate, furazolidone, quinine, spiramycin, alpha-difluoromthyl-ornithine, interleukin-2 and the like. Representative agents useful in combination with the compounds of the invention for the treatment of *Helicobacter* include, for example, azithromycin, amoxycillin, clarithromycin and the like. The compounds of the invention may also be administered in combination with β-lactams, such as cephalosporins, carbapenems and/or monobactams, or other agents useful in the treatment of pneumonia.

The above compounds to be employed in combination with the compounds of the invention will be used in therapeutic amounts as indicated in the PHYSICIANS' DESK REFERENCE (PDR) 47th Edition (1993), which is incorporated herein by reference, or such therapeutically useful amounts as would be known to one of ordinary skill in the art.

The compounds of the invention and the other antiinfective agent can be administered at the recommended maximum clinical dosage or at lower doses. Dosage levels of the active compounds in the compositions of the invention may be varied so as to obtain a desired therapeutic response depending on the route of administration, severity of the disease and the response of the patient. The combination can be administered as separate compositions or as a single dosage form containing both agents. When administered as a combination, the therapeutic agents can be formulated as separate compositions, which are given at the same time or different times, or the therapeutic agents, can be given as a single composition.

The foregoing may be better understood from the following examples, which are presented for the purposes of illustration and are not intended to limit the scope of the inventive concepts.

### Example 1

### P. Aeruginosa Susceptibility

### Strains and Cultures

Bacterial isolates were cultivated from -70°C frozen stocks by two consecutive overnight passages at 37°C on 5% sheep blood agar. Clinical isolates of *P. aeruginosa* and *Burkholderia cepacia* were obtained from CF centers that participated in the TOBI® phase 3 clinical trials. Laboratory strain *P. aeruginosa* ATCC #27853 was used as the quality control strain for MIC and MBC testing. It was also the test organism in the mucin MBC assay, and the time-kill assays.

### Susceptibility

Determinations of minimum inhibitory concentration (MIC) and minimum bactericidal concentration (MBC) were performed by broth microdilution, in accordance with NCCLS guidelines (*National Committee for Clinical Laboratory Standards.* 1997. Methods for Dilution Antimicrobial Susceptibility Tests for Bacteria That Grow Aerobically-Fourth Edition; Approved Standard. Document M7-A4, Vol. 17, no. 2, Villanova, PA. 10-15; and *National Committee for Clinical Laboratory Standards.* 1992. Methods for Determining Bactericidal Activity of Antimicrobial Agents - Tentative Guideline. Document M26T, Vol. 12, no.19, Villanova, PA. 8-19), for 7 *P. aeruginosa* CF clinical isolates. Serial 2-fold dilutions of PA-1806 and other comparator compounds as needed were prepared in 96-well sterile polystyrene broth microdilution plates. These plates were inoculated with standardized (between 1x10⁵ and 1x10⁶ CFU/mL) suspensions of the test strains. The drug dilutions and standardized bacterial inocula were created in sterile Cation Adjusted Mueller-Hinton Broth (CAMH). Following overnight incubation at 37°C, the MIC was defined, by visual examination, as the lowest concentration of a given antibiotic that prevented development of visible growth of the test strain.

The MBC was determined from the MIC broth microdilution plate by subculture of individual wells devoid of visible turbidity (at or above the MIC). A ten microliter volume from each well was subcultured onto an SBA plate, and the colonies counted after overnight incubation at 37°C. The MBC value is the lowest antibiotic concentration that killed 99.9% of the initial inoculum.

The results are shown in the following Table 1:

**Table 1**

| | | PA-1806 | | |
|---|---|---|---|---|
| Isolate # | Mucoid Phenotype? | MIC(µg/mL) | MBC(µg/mL) | MBC/MIC Ratio |
| 449 | Mucoid | 0.25 | 0.25a | 1 |
| 918 | Mucoid | 0.13 | 1a | 8 |
| 1585 | Non-Mucoid | 1 | 8 | 8 |
| 3140 | Mucoid | 0.13b | 2 | 16 |
| 3152 | Mucoid | 0.03b | 16 | 512 |
| 3200 | Non-Mucoid | 0.13b | 4a | 32 |
| 3288 | Non-Mucoid | 16 | >256 | 16 |
| #27853(QC) | Non-Mucoid | 0.25 | 0.25 | 1 |

### Example 2

### Time Kill Kinetics

Time-kill kinetic assays of PA-1806 were performed against *P.aeruginosa* ATCC 27853, and a clinical isolate of *B. cepacia* (#3901B). PA-1806 was tested at the MBC, and multiples of the MBC. The bacterial inoculum was prepared and standardized as previously described for MIC testing. Several glass screw-capped tubes, each containing a 10 mL aliquot of bacterial inoculum, were prepared. Antibiotic was added to each tube to create the desired final concentrations, and one tube was left untreated to serve as a growth control. The tubes were then incubated at 37°C in rotating fashion. At time zero and at subsequent timepoints, 200 µL aliquots were removed from the tubes, diluted in sterile saline, and subcultured in duplicate on SBA plates. The inoculated SBA plates were incubated overnight at 37°C, and the colonies counted. Time-kill kinetics were expressed as the elapsed time needed to produce a 3 log reduction in CFU/mL count, as compared to the initial inoculum (see MBC, described above) at a given concentration of antibiotic. Thus, the bactericidal concentration displays a 3 log reduction in bacterial count compared to initial inoculum CFU/mL count

### Mucin Effect on Time-Kill Assays

Previous research demonstrated that the activity of some antibiotics is antagonized by CF patient sputum, which decreases the resultant antibacterial effect (Mendelman, P.M., A.L. Smith, J. Levy, et al. 1985. Aminoglycoside penetration, inactivation, and efficacy in cystic fibrosis sputum. *Am. Rev. Respir. Dis.* **132:**761-765. Mucin binding of PA-1806 and tobramycin was tested by the addition of gastric mucin (2% final volume) to the MH broth used to set up the time-kill assay described above. The same *P. aeruginosa* strain was evaluated in both formats. Mucin was deemed to have a significant effect on activity if the time-kill cidal concentration increased by 4-fold or greater over the results obtained with plain MH broth.

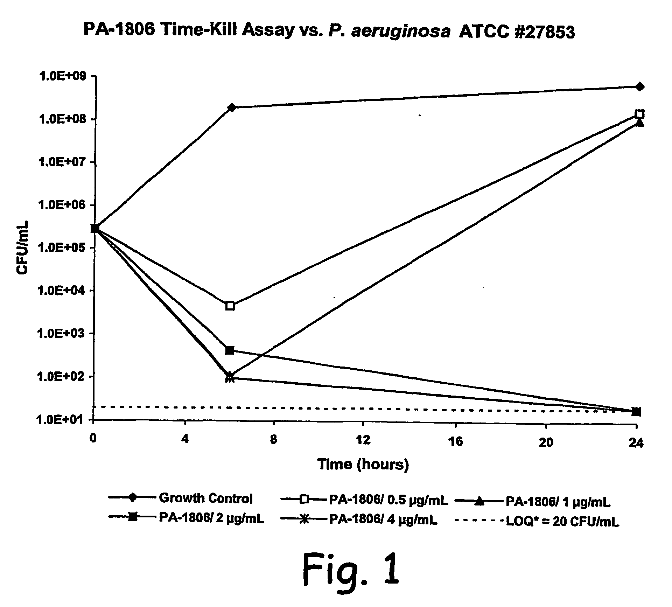

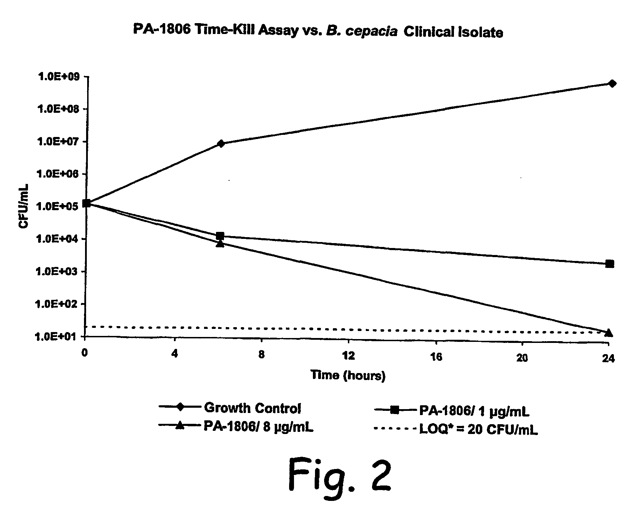

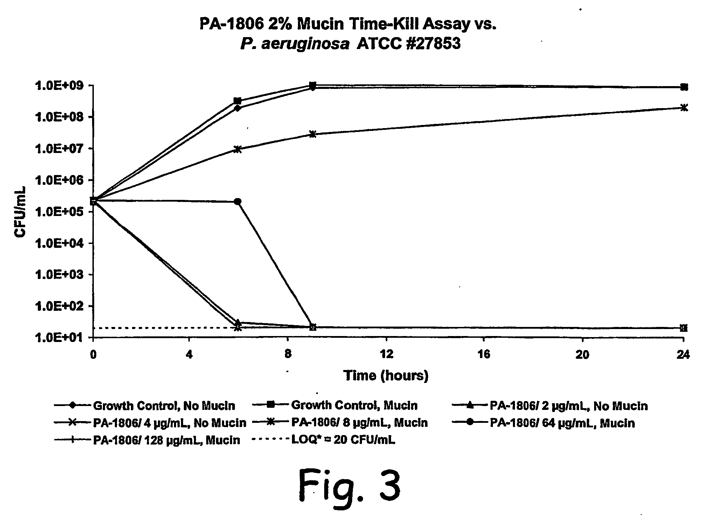

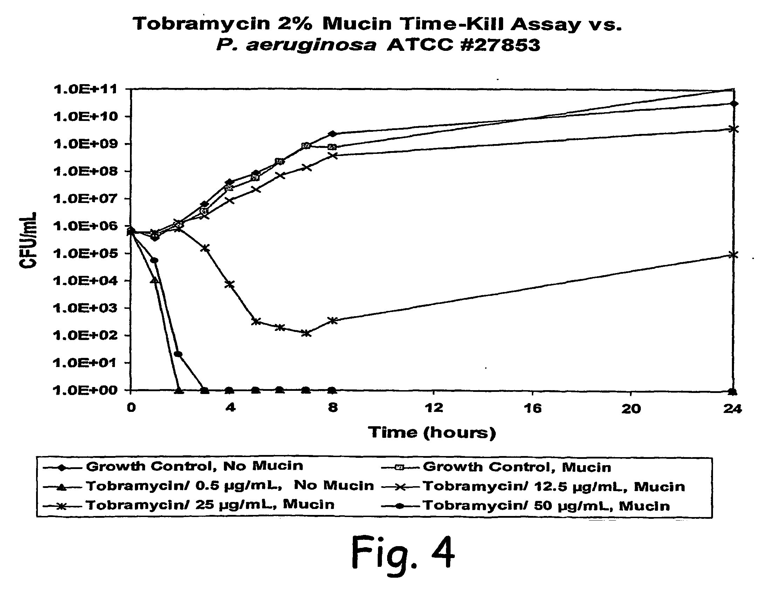

The results are shown in Figures 1-4. Generally, PA-1806 shows good bactericidal activity against *P. aeruginosa* ATCC 27853. However, the compound did not demonstrate bactericidal activity against a small group of P. aeruginosa CF clinical isolates. The time-kill assay against P. aeruginosa ATCC 27853 in standard format showed PA-1806 to be bactericidal at 4 mg/mL by the 6 hour timepoint, and at 2 mg/mL by the 24 hour timepoint The time-kill assay against the *B. cepacia* clinical isolate in standard format showed PA-1806 to be bactericidal at 8 mg/mL by the 24 hour timepoint. The time-kill assay in the presence of 2% mucin showed PA-1806 to be bactericidal by 24 hours at a concentration of 64mg/mL (32X the standard format MBC). The performance of PA-1806 was adversely affected by mucin. However, similar effects were observed for tobramycin. Mucin time-kill assays using PA-1806 and tobramycin suggest that co-administration of a mucolytic agent will overcome this effect.

### Example 3

### In Vivo Administration

A patient suffering from CF and presenting an advanced *P. aeruginosa* infection in the lungs is treated with N-acetyl-L-cysteine (MUCOSIL™; Dey Laboratories) in accordance with the instructions of the manufacturer's product insert. After 5 minutes, the patient is treated by inhalation with a dry powder formulation of 100 mg of PA-1806 over a period of 10 minutes. A decrease in the level of *P. aeruginosa* infection in the lungs of the patient is observed.

## Claims

1. Use of an antibacterial monobactam compound, or a pharmaceutically acceptable salt or prodrug thereof, and an amount of a mucolytic agent effective to enhance the antibacterial activity of the monobactam compound in the endobronchial space of the patient in the manufacture of a medicament for the treatment of a patient suffering from a pathogenic endobronchial microbial infection, wherein the mucolytic agent may be administered to the patient prior to, simulatenously with or after administration of the antibacterial monobactam compound.

2. A use of Claim 1 wherein the nionobactam compound is [2R [2α, 3α(Z)]]-3-[[[[1 (2 amino-4-thiazolyl)-2 [(2-methyl 4-oxo-1 sulfo-3 azetidinyl)amino]-2 oxoethylidene]ainino]oxy]methyl]-6,7-dihydroxy-2-quinoxalinecarboxylic acid, aztreonam or carumonam, or a pharmaceutically acceptable salt or prodrug thereof.

3. A use of Claim 1 wherein the mucolytic agent is selected from the group consisting of N-acetyl-L-cysteine, recombinant human Dnase, Erdosteine, Gauifenesin, and sodium taurocholate.

4. A use of Claim 1 wherein the mucolytic agent is administered to the patient prior to administration of the monobactam compound.

5. A method for inhibiting the growth of susceptible microbes *in vitro* in the presence of mucin by treating the microbes with a antibacterially effective amount of an monobactam compound, or a pharmaceutically acceptable salt or prodrug thereof, and with an amount of a mucolytic agent effective to enhance the antibacterial activity of the monobactam compound

6. A method of Claim 5 wherein the monobactam compound is [2R [2α, 3α(Z)]]-3-[[[[1 (2 amino-4-thiazolyl)-2 [(2 methyl 4-oxo-1 sulfo-3 azetidinyl)amino]-2 oxoethylidene]amino]oxy]methyl]-6,7-dihydroxy-2-quinoxalinecarboxylic acid, aztreonam or carumonam, or a pharmaceutically acceptable salt or prodrug thereof.

7. A method of Claim 5 wherein the mucolytic agent is selected from the group consisting of N-acetyl-L-cysteine, recombinant human Dnase, Erdosteine, Gauifenesin, and sodium taurocholate.

8. A pharmaceutical composition for treating patients suffering from a pathogenic endobronchial microbial infection, comprising an antibacterially effective amount of monobactam compound, or a pharmaceutically acceptable salt or prodrug thereof, and an amount of a mucolytic agent effective to enhance the antibacterial activity of the monobactam compound when administered to the endobronchial space of a patient.

9. A composition of Claim 8 wherein the monobactam compound is [2R [2α, 3α(Z)]]-3-[[[[1(2 amino-4-thiazolyl)-2 [(2 methyl 4-oxo-1 sulfo-3 azetidinyl)amino]-2-oxoethylidene]amino]oxy]methyl]-6,7-dihydroxy-2-quinoxalinecarboxylic acid, aztreonam or carumonam, or a pharmaceutically acceptable salt or prodrug thereof.

10. A composition of Claim 8 wherein the mucolytic agent is selected from the group consisting of N-acetyl-L-cysteine, recombinant human Dnase, Erdosteine, Gauifenesin, and sodium taurocholate.

11. A therapeutic package suitable for commercial sale for treating a patient suffering from a pathogenic endobronchial microbial infection, comprising a container, a therapeutically effective amount of an antibacterial monobactam compound, or a pharmaceutically acceptable salt or prodrug thereof, and an amount of a mucolytic agent effective to enhance the antibacterial activity of the monobactam compound in the endobronchial space of the patient.

12. A therapeutic package of claim 11 further comprising written matter instructing that the patient receive treatment with the mucolytic agent prior to treatment with the antibacterial monobactam compound.

13. A therapeutic package of claim 11 wherein the monobactam compound is [2R[2α, 3α(Z)]]-3-[[[[1 (2 amino-4-thiazolyl)-2 [(2 methyl 4-oxo-1 sulfo-3 azetidinyl)amino]-2 oxoethylidene]amino]oxy]methyl]-6,7-dihydroxy-2-quinoxalinecarboxylic acid, aztreonam or carumonam, or a pharmaceutically acceptable salt or prodrug thereof.

14. A therapeutic package of claim 13 wherein the mucolytic agent is selected from the group consisting of N-acetyl-L-cysteine, recombinant human Dnase, Erdosteine, Gauifenesin, and sodium taurocholate.

15. A therapeutic package of claim 11 further comprising a nebulizer.

## Patentansprüche

1. Verwendung einer antibakteriellen Monobactam-Verbindung oder eines pharmazeutisch verträglichen Salzes oder einer Vorstufe (prodrug) derselben sowie einer Menge eines mucolytischen Mittels, die wirksam ist, die antibakterielle Aktivität der Monobactam-Verbindung im Endobronchialraum des Patienten zu verstärken, bei der Herstellung eines Medikamentes zur Behandlung eines Patienten, der an einer pathogenen endobronchialen mikrobiellen Infektion leidet, wobei das mucolytische Mittel dem Patienten vor, gleichzeitig mit oder nach der Verabreichung der antibakteriellen Monobactam-Verbindung verabreicht werden kann.

2. Verwendung nach Anspruch 1, bei der die Monobactam-Verbindung [2R[2α,3α(Z)]]-3-[[[[1(2-Amino-4-thiazolyl)-2[(2-methyl-4-oxo-1-sulfo-3-azetidinyl)amino]-2-oxoethyliden]amino]oxy]methyl]-6,7-dihydroxy-2-chinoxalincarboxylsäure, Aztreonam oder Carumonam oder ein pharmazeutisch verträgliches Salz oder eine Vorstufe (prodrug) derselben ist.

3. Verwendung nach Anspruch 1, bei der das mucolytische Mittel ausgewählt ist aus der Gruppe bestehend aus N-Acetyl-L-cystein, rekombinanter humaner Dnase, Erdostein, Gauifenesin und Natriumtaurocholat.

4. Verwendung nach Anspruch 1, bei der das mucolytische Mittel dem Patienten vor der Verabreichung der Monobactam-Verbindung verabreicht wird.

5. Verfahren zur Hemmung des Wachstums von empfindlichen Mikroben *in vitro* in Gegenwart von Mucin durch Behandlung der Mikroben mit einer antibakteriell wirksamen Menge einer Monobactam-Verbindung oder eines pharmazeutisch verträglichen Salzes oder einer Vorstufe (prodrug) derselben sowie mit einer Menge eines mucolytischen Mittels, die wirksam ist, die antibakterielle Aktivität der Monobactam-Verbindung zu verstärken.

6. Verfahren nach Anspruch 5, bei der die Monobactam-Verbindung [2R[2α,3α(Z)]]-3-[[[[1(2-Amino-4-thiazolyl)-2[(2-methyl-4-oxo-1-sulfo-3-azetidinyl)amino]-2-oxoethyliden]amino]oxy]methyl]-6,7-dihydroxy-2-chinoxalincarboxylsäure, Aztreonam oder Carumonam oder ein pharmazeutisch verträgliches Salz oder eine Vorstufe (prodrug) derselben ist.

7. Verfahren nach Anspruch 5, bei dem das mucolytische Mittel ausgewählt ist aus der Gruppe bestehend aus N-Acetyl-L-cystein, rekombinanter humaner Dnase, Erdostein, Gauifenesin und Natriumtaurocholat.

8. Pharmazeutische Zusammensetzung zur Behandlung von Patienten, die an einer pathogenen endobronchialen mikrobiellen Infektion leiden, wobei die Zusammensetzung eine antibakteriell wirksame Menge einer Monobactam-Verbindung oder eines pharmazeutisch verträglichen Salzes oder einer Vorstufe (prodrug) derselben sowie eine Menge eines mucolytischen Mittels umfasst, die wirksam ist, die antibakterielle Aktivität der Monobactam-Verbindung zu verstärken, wenn diese in den Endobronchialraum eines Patienten verabreicht wird.

9. Verbindung nach Anspruch 8, bei der die Monobactam-Verbindung [2R[2α,3α(Z)]]-3-[[[[1(2-Amino-4-thiazolyl)-2[(2-methyl-4-oxo-1-sulfo-3-azetidinyl)amino]-2-oxoethyliden]amino]oxy]methyl]-6,7-dihydroxy-2-chinoxalincarboxylsäure, Aztreonam oder Carumonam oder ein pharmazeutisch verträgliches Salz oder eine Vorstufe (prodrug) derselben ist.

10. Verbindung nach Anspruch 8, bei der das mucolytische Mittel ausgewählt ist aus der Gruppe bestehend N-Acetyl-L-cystein, rekombinanter humaner Dnase, Erdostein, Gauifenesin und Natriumtaurocholat.

11. Therapeutisches Paket, das für den kommerziellen Verkauf zur Behandlung eines Patienten geeignet ist, der an einer pathogenen endobronchialen mikrobiellen Infektion leidet, wobei das Paket einen Behälter, eine therapeutisch wirksame Menge einer antibakteriellen Monobactam-Verbindung oder eines pharmazeutisch verträglichen Salzes oder einer Vorstufe (prodrug) derselben sowie eine Menge eines mucolytischen Mittels umfasst, die wirksam ist, die antibakterielle Aktivität einer Monobactam-Verbindung im Endobronchialraum des Patienten zu verstärken.

12. Therapeutisches Paket nach Anspruch 11, das ferner schriftliche Anweisungen umfasst, dass der Patient eine Behandlung mit dem mucolytischen Mittel vor der Behandlung mit der antibakteriellen Monobactam-Verbindung erhält.

13. Therapeutisches Paket nach Anspruch 11, bei der die Monobactam-Verbindung [2R[2α,3α(Z)]]-3-[[[[1(2-Amino-4-thiazolyl)-2[(2-methyl-4-oxo-1-sulfo-3-azetidinyl)amino]-2-oxoethyliden]amino]oxy]methyl]-6,7-dihydroxy-2-chinoxalincarboxylsäure, Aztreonam oder Carumonam oder ein pharmazeutisch verträgliches Salz oder eine Vorstufe (prodrug) derselben ist.

14. Therapeutisches Paket nach Anspruch 13, bei der das mucolytische Mittel ausgewählt ist aus der Gruppe bestehend N-Acetyl-L-cystein, rekombinanter humaner Dnase, Erdostein, Gauifenesin und Natriumtaurocholat.

15. Therapeutisches Paket nach Anspruch 11, das ferner einen Zerstäuber umfasst.

## Revendications

1. Utilisation d'un composé monobactame antibactérien ou d'un sel pharmaceutiquement acceptable ou d'un promédicament de celui-ci, et d'une quantité efficace d'un agent mucolytique pour accroître l'activité antibactérienne du composé monobactame dans l'espace endobronchique du patient dans la fabrication d'un médicament pour le traitement d'un patient souffrant d'une infection microbienne endobronchique pathogène, dans laquelle l'agent mucolytique peut être administré au patient avant, simultanément à ou après l'administration du composé monobactame antibactérien.

2. Utilisation selon la revendication 1 dans laquelle le composé monobactame est l'acide [2R [2α, 3 α(Z)]]-3-[[[[1 (2 amino-4-thiazolyl)-2 [(2 méthyl 4-oxo-1 sulfo-3 azétidinyl)amino]-2oxoéthylidène]amino]oxy]méthyl]-6,7-dihydroxy-2-quinoxalinecarboxylique, l'aztréonam ou le carumonam ou un sel pharmaceutiquement acceptable ou un promédicament de celui-ci.

3. Utilisation selon la revendication 1 dans laquelle l'agent mucolytique est choisi dans le groupe consistant en la N-acétyl-L-cystéine, la désoxyribonucléase recombinante humaine, l'Erdostéine, Guaifenesin et le taurocholate de sodium.

4. Utilisation selon la revendication 1 dans laquelle l'agent mucolytique est administré au patient avant administration du composé monobactame.

5. Procédé pour inhiber la croissance des microbes sensibles *in vitro,* en présence de mucine, en traitant les microbes avec une quantité efficace en terme d'activité antibactérienne d'un composé monobactame ou d'un sel pharmaceutiquement acceptable ou d'un promédicament de celui-ci et avec une quantité efficace d'un agent mucolytique pour accroître l'activité antibactérienne du composé monobactame.

6. Procédé selon la revendication 5 dans laquelle le composé monobactame est l'acide [2R [2α, 3 α(Z)]]-3-[[[[1 (2 amino-4-thiazolyl)-2 [(2 méthyl 4-oxo-1 sulfo-3 azétidinyl)amino]-2oxoéthylidène]amino]oxy]méthyl]-6,7-dihydroxy-2-quinoxalinecarboxylique, l'aztréonam ou le carumonam ou un sel pharmaceutiquement acceptable ou un promédicament de celui-ci.

7. Procédé selon la revendication 5 dans laquelle l'agent mucolytique est choisi dans le groupe consistant en la N-acétyl-L-cystéine, la désoxyribonucléase recombinante humaine, l'Erdostéine, Guaifenesin et le taurocholate de sodium.

8. Composition pharmaceutique pour traiter des patients souffrant d'une infection microbienne endobronchique pathogène, comprenant une quantité efficace en terme d'activité antibactérienne d'un composé monobactame ou d'un sel pharmaceutiquement acceptable ou d'un promédicament de celui-ci et une quantité efficace d'un agent mucolytique pour accroître l'activité antibactérienne du composé monobactame quand elle est administrée dans l'espace endobronchique d'un patient.

9. Composition selon la revendication 8 dans laquelle le composé monobactame est l'acide [2R [2α, 3 α(Z)]]-3-[[[[1 (2 amino-4-thiazolyl)-2 [(2 méthyl 4-oxo-1 sulfo-3 azétidinyl)amino]-2oxoéthylidène]amino]oxy]méthyl]-6,7-dihydroxy-2-quinoxalinecarboxylique, l'aztréonam ou le carumonam ou un sel pharmaceutiquement acceptable ou un promédicament de celui-ci.

10. Composition selon la revendication 8 dans laquelle l'agent mucolytique est choisi dans le groupe consistant en la N-acétyl-L-cystéine, la désoxyribonucléase recombinante humaine, l'Erdostéine, Guaifenesin et le taurocholate de sodium.

11. Conditionnement thérapeutique approprié à la vente commerciale pour traiter un patient souffrant d'une infection microbienne endobronchique pathogène, comprenant un récipient, une quantité thérapeutiquement efficace d'un composé monobactame antibactérien ou d'un sel pharmaceutiquement acceptable ou d'un promédicament de celui-ci et une quantité efficace d'un agent mucolytique pour accroître l'activité antibactérienne du composé monobactame dans l'espace endobronchique du patient.

12. Conditionnement thérapeutique selon la revendication 11 comprenant en outre une instruction écrite informant que le patient reçoit le traitement avec l'agent mucolytique avant le traitement avec le composé monobactame antibactérien.

13. Conditionnement thérapeutique selon la revendication 11 dans laquelle le composé monobactame est l'acide [2R [2α, 3 α(Z)]]-3-[[[[1 (2 amino-4-thiazolyl)-2 [(2 méthyl 4-oxo-1 sulfo-3 azétidinyl)amino]-2 oxoéthylidène]amino]oxy]méthyl]-6,7-dihydroxy-2-quinoxalinecarboxylique, l'aztréonam ou le carumonam ou un sel pharmaceutiquement acceptable ou un promédicament de celui-ci.

14. Conditionnement thérapeutique selon la revendication 13 dans laquelle l'agent mucolytique est choisi dans le groupe consistant en la N-acétyl-L-cystéine, la désoxyribonucléase recombinante humaine, l'Erdostéine, Guaifenesin et le taurocholate de sodium.

15. Conditionnement thérapeutique selon la revendication 11 comprenant en outre un nébulisateur.
